# EUROPEAN PATENT APPLICATION

(11) **EP 3 564 958 A1**
(43) Date of publication of application: **06.11.2019**
(21) Application number: 19176139.4
(22) Date of filing: 28.05.2014
(51) Int. Cl.: G16B 5/20

(54) **PARADIGM DRUG RESPONSE NETWORKS**

(30) Priority: 28.05.2013 US 201361828145 P; 20.12.2013 US 201361919289 P
(62) Divisional of application: 14804788.9
(71) Applicant: Five3 Genomics, LLC, Santa Cruz, California 95060 (US)
(72) Inventor: BENZ, Stephen Charles, Santa Cruz, CA California 95062 (US); SZETO, Christopher, Santa Cruz, CA California 95060 (US)
(74) Representative: Elkington and Fife LLP

(57) **Abstract**

Systems and methods are presented in which omics data from multiple cell or tissue samples are used to identify pathway elements that are associated with a treatment parameter of the cell or tissue (*e.g.,* resistance towards a specific drug). So identified pathway elements are then modulated *in silico* in a statistical factor graph model to provide a modified data set that is re-evaluated with respect to the treatment parameter. Such systems and models are particularly useful for recommendation of multi-drug treatments for treatment-naive patients.

## Description

### Field of The Invention

The field of the invention is computational modeling and use of pathway models, especially as it relates to *in silico* modulation of pathway models to identify pathway elements useful for development of treatment recommendations.

### Background

The background description includes information that may be useful in understanding the present invention. It is not an admission that any of the information provided herein is prior art or relevant to the presently claimed invention, or that any publication specifically or implicitly referenced is prior art.

Various systems and methods of computational modeling of pathways are known in the art. For example, some algorithms (e.g., GSEA, SPIA, and PathOlogist) are capable of successfully identifying altered pathways of interest using pathways curated from literature. Still further tools have constructed causal graphs from curated interactions in literature and used these graphs to explain expression profiles. Algorithms such as ARACNE, MINDy and CONEXIC take in gene transcriptional information (and copy-number, in the case of CONEXIC) to so identify likely transcriptional drivers across a set of cancer samples. However, these tools do not attempt to group different drivers into functional networks identifying singular targets of interest. Some newer pathway algorithms such as NetBox and Mutual Exclusivity Modules in Cancer (MEMo) attempt to solve the problem of data integration in cancer to thereby identify networks across multiple data types that are key to the oncogenic potential of samples.

While such tools allow for at least some limited integration across pathways to find a network, they generally fail to provide regulatory information and association of such information with one or more effects in the relevant pathways or network of pathways. Likewise, GIENA looks for dysregulated gene interactions within a single biological pathway but does not take into account the topology of the pathway or prior knowledge about the direction or nature of the interactions. Moreover, due to the relative incomplete nature of these modeling systems, predictive analysis is often impossible, especially where interactions of multiple pathways and/or pathway elements are under investigation.

More recently, various improved systems and methods have been described to obtain *in silico* pathway models of *in vivo* pathways, and exemplary systems and methods are described in WO 2011/139345 and WO 2013/062505. Further refinement of such models was provided in WO 2014/059036 (collectively referred to herein as "PARADIGM") disclosing methods to help identify cross-correlations among different pathway elements and pathways. While such models provide valuable insights, for example, into interconnectivities of various signaling pathways and flow of signals through various pathways, numerous aspects of using such modeling have not been appreciated or even recognized.

All publications identified herein are incorporated by reference to the same extent as if each individual publication or patent application were specifically and individually indicated to be incorporated by reference. Where a definition or use of a term in an incorporated reference is inconsistent or contrary to the definition of that term provided herein, the definition of that term provided herein applies and the definition of that term in the reference does not apply. Thus, there is still a need to provide improved computational models and methods to predict *in silico* response of one or more pathways in a diseased cell or tissue to a simulated condition (e.g., simulated therapeutic intervention) to so help predict a desired therapeutic outcome.

### Summary of The Invention

The present inventive subject matter is directed to devices, systems, and methods for *in silico* prediction of a therapeutic outcome using omics data obtained from a patient sample and *a priori* pathway models. In preferred aspects, prediction of therapeutic outcomes is based on *in silico* modulation of a pathway model to simulate a therapeutic approach, and the outcome of the simulation is employed to prepare a treatment recommendation.

In one aspect of the inventive subject matter, the inventors therefore contemplate a method of *in silico* analysis of data sets derived from omics data of cells. Preferred methods particularly include a step of informationally coupling a pathway model database to a machine learning system and a pathway analysis engine, wherein the pathway model database stores multiple distinct data sets derived from omics data of multiple distinct diseased cells, respectively, and wherein each data set comprises a plurality of pathway element data. The machine learning system then receives at least some of the plurality of distinct data sets and identifies a determinant pathway element in the distinct data sets that is associated with a status (*e.g.*, sensitive or resistant) of a treatment parameter (*e.g.,* treatment with a drug) of the diseased cells. In a further step, the pathway analysis engine then receives at least one of the distinct data sets from the diseased cells, and the determinant pathway element in the data set is then modulated in the pathway analysis engine to so produce a modified data set. The machine learning system then uses the modified data set to identify a change in status of the treatment parameter for the diseased cell. Where desirable or needed, it is contemplated that the systems and methods herein will also include an additional step of pre-processing the datasets (*e.g.*, feature selection, data transformation, metadata transformation, and/or splitting into training and validation datasets).

Most typically, at least one of the distinct data sets is generated from a patient sample of a patient diagnosed with a neoplastic disease, while one or more other data sets are generated from distinct cell cultures containing cells that are not from the patient. It should be noted that cells from the cell cultures are of the same neoplastic type as the neoplastic disease of the patient (*e.g*., various breast cancer cell lines not derived from the patient and breast cancer cells or tissue). Furthermore, it should be appreciated that the patient will not have been treated for the neoplastic disease. Viewed from another perspective, contemplated systems and methods are suitable to predict drug combinations suitable for optimized outcome based on patient omics data before treatment even commences. While not limiting to the inventive subject matter, it is generally preferred that output data are generated that comprise a treatment recommendation for the patient. Thus, contemplated methods will also include a step of identifying a drug that targets the determinant pathway element when the change in status exceeds a predetermined threshold.

Viewed from a different perspective, it should be appreciated that the plurality of distinct diseased cells will differ from one another with respect to sensitivity of the cells to a drug (or other treatment modality, including radiation, heat treatment, etc.). For example, a first set of the distinct diseased cells may be sensitive to treatment with a drug, while a second set of the distinct diseased cells may be resistant to treatment with the drug.

With respect to omics data, all known omics data are considered suitable and preferred omics data especially include gene copy number data, gene mutation data, gene methylation data, gene expression data, RNA splice information data, siRNA data, RNA translation data, and/or protein activity data. Likewise, numerous data formats are deemed appropriate for use herein, however, particularly preferred data formats are PARADIGM datasets. Determinant pathway element may vary considerably, however, especially preferred determinant pathway elements include the expression state of a gene, the protein level of a protein, and/or protein activity of a protein.

Therefore, the inventors also contemplate a system for *in silico* analysis of data sets derived from omics data of cells that will include a pathway model database that is informationally coupled to a machine learning system and a pathway analysis engine. Most typically, the pathway model database will be programmed to store a plurality of distinct data sets derived from omics data of a plurality of distinct diseased cells, respectively, and each data set will comprise a plurality of pathway element data. The machine learning system is then programmed to receive from the pathway model database the plurality of distinct data sets, and further programmed to identify a determinant pathway element in the plurality of distinct data sets that is associated with a status of a treatment parameter of the diseased cells. Most typically, the pathway analysis engine is programmed to receive at least one of the distinct data sets from the diseased cells and further programmed to modulate the determinant pathway element in the at least one distinct data set to produce a modified data set from the diseased cell, and the machine learning system is programmed to identify a change in the status of the treatment parameter for the diseased cell using the modified data set. Typically, the system is further programmed to generate output data that comprise a treatment recommendation for the patient.

As noted above, it is also contemplated that at least one of the distinct data sets is generated from a patient sample of a patient having a neoplastic disease, and that multiple other ones of the distinct data sets are generated from distinct cell cultures containing cells that are not from the patient. Preferably, the patient has not been treated for the neoplastic disease. Viewed form a different perspective, the inventors also contemplate a non-transient computer readable medium containing program instructions for causing a computer system in which a pathway model database is coupled to a machine learning system and a pathway analysis engine to perform a method that comprises the steps of (a) transferring from the pathway model database to the machine learning system a plurality of distinct data sets derived from omics data of a plurality of distinct diseased cells, respectively, and wherein each data set comprises a plurality of pathway element data; (b) identifying, by the machine learning system, a determinant pathway element in the plurality of distinct data sets that is associated with a status of a treatment parameter of the diseased cells; (c) receiving, by the pathway analysis engine, at least one of the distinct data sets from the diseased cells; (d) modulating, by the pathway analysis engine, the determinant pathway element in the at least one distinct data set to produce a modified data set from the diseased cell; and (e) identifying, by the machine learning system and using the modified data set, a change in the status of the treatment parameter for the diseased cell.

Most typically, the omics data may include gene copy number data, gene mutation data, gene methylation data, gene expression data, RNA splice information data, siRNA data, RNA translation data, and/or protein activity data, and it is especially contemplated that the distinct data sets are PARADIGM datasets.

Various objects, features, aspects and advantages of the inventive subject matter will become more apparent from the following detailed description of preferred embodiments, along with the accompanying drawing figures in which like numerals represent like components.

### Brief Description of the Drawing

Figures 1A and 1B depict sensitivity of breast cancer cell lines against selected drugs (1A Cisplatin; 1B Geldanamycin) in the left panels, and schematically depicts the activity of pathway elements in these cell lines related to the selected drugs in the right panels.
Figure 1C depicts sensitivity of a variety of breast cancer cell lines against Cisplatin as expressed in GI₅₀ (upper panel) and corresponding heat map for gene expression/regulation for the same cells (lower panel).
Figure 2A schematically illustrates a pathway model system in which each gene is represented via a statistical factor graph model.
Figure 2B schematically represents an *in silico* modulation of a pathway element of Figure 2A and associated downstream effects.
Figure 2C schematically illustrates a pharmaceutical intervention simulation in an exemplary pathway modeling system.
Figure 2D schematically illustrates significance analysis and shift measurement according to the inventive subject matter.
Figure 3 schematically illustrates an *in vivo* validation experiment for *in silico* knock-down of a gene in a colon cancer cell line.
Figure 4 is a schematic illustration of a workflow according to the inventive subject matter.
Figure 5A is an exemplary output for predicted changes in cisplatin sensitivity after *in silico* manipulation of various cancer cell lines in which IGFBP2 was knocked out.
Figure 5B is an exemplary output for predicted changes in GSK923295 sensitivity after *in silico* manipulation of various cancer cell lines in which TP53INP1 was knocked out.
Figure 5C is an exemplary output for predicted changes in Fascaplysin sensitivity after *in silico* manipulation of various cancer cell lines in which ARHGEF25 was knocked out.

### Detailed Description

Based on recently developed pathway analysis systems and methods as described in more detail in WO 2011/139345, WO/2013/062505, and WO/2014/059036, incorporated by reference herein, the inventors now contemplate that pathway analysis and pathway model modifications can be used *in silico* to identify drug treatment options and/or simulate drug treatment targeting pathway elements that are a determinant of or associated with a treatment-relevant parameter (*e.g.,* drug resistance and/or sensitivity to a particular treatment) of a condition, and especially a neoplastic disease.

More specifically, identified pathway elements are modulated or modified *in silico* using a pathway analysis system and method to test if a desired effect could be achieved. For example, where a pathway model for drug resistance identifies over-expression of a certain element as critical to development of a condition (*e.g.*, drug resistance against a particular drug), expression level of that element could be reduced *in silico* to thereby test in the same pathway analysis system and method if reduction of that element *in silico* could potentially reverse the cell to drug sensitivity. Such approach is particularly valuable where multiple cell lines representing multiple possible tumor variants are already available. In such a case, pathway analysis can be performed for each of the cell lines to so obtain a collection of cell line-specific pathway models. Such collection is particularly useful for comparison with data obtained from a patient sample, as the data for patient sample can be analyzed within the same data space as the collection, which ultimately allows for identification of treatment targets for the patient. Among other advantages, contemplated systems and methods therefore allow analysis of patient data from a tumor sample to identify multi-drug treatment before the patient has actually undergone the drug treatment.

Therefore, and viewed from a different perspective, the inventors have discovered that various omics data from diseased cells and/or tissue of a patient can be used in a computational approach to determine a sensitivity profile for the cells and/or tissue, wherein the profile is based on *a priori* identification of pathways and/or pathway elements in a variety of similarly diseased cells (*e.g.,* breast cancer cells). Most preferably, the *a priori* identified pathway(s) and/or pathway element(s) are associated with the resistance and/or sensitivity to a particular pharmaceutical intervention and/or treatment regimen. Once the sensitivity profile is established, treatment can be directly predicted from the *a priori* identified pathway(s) and/or pathway element(s), or identified pathways and/or pathway elements can be modulated *in silico* using known pathway modeling system and methods to so help predict likely outcomes for the pharmaceutical intervention and/or treatment regimen.

It should be noted that any language directed to a computer should be read to include any suitable combination of computing devices, including servers, interfaces, systems, databases, agents, peers, engines, controllers, or other types of computing devices operating individually or collectively. One should appreciate the computing devices comprise a processor configured to execute software instructions stored on a tangible, non-transitory computer readable storage medium (e.g., hard drive, solid state drive, RAM, flash, ROM, etc.). The software instructions preferably configure the computing device to provide the roles, responsibilities, or other functionality as discussed below with respect to the disclosed apparatus. In especially preferred embodiments, the various servers, systems, databases, or interfaces exchange data using standardized protocols or algorithms, possibly based on HTTP, HTTPS, AES, public-private key exchanges, web service APIs, known financial transaction protocols, or other electronic information exchanging methods. Data exchanges preferably are conducted over a packet-switched network, the Internet, LAN, WAN, VPN, or other type of packet switched network.

Most cancer patients are rarely subject to monotherapy, however, accurate prediction of a response to particular drug combinations is one of the most profound challenges in cancer therapy. As the number of potential drug combinations is large, there is currently little statistically significant data to support any given combination for a specific cancer. Instead, most of the current combination therapies are hand-selected to target independent pathways. Unfortunately, while current methods to design combination therapies are somewhat pragmatic, they tend to be perfunctory as there is no accurate statistical approach to identify candidate drugs for synergistic dual therapy. Moreover, numerically combining monotherapy predictions will not accurately predict the results of combinations, as the mechanisms of drug response are not necessarily independent.

To address this shortcoming, the inventors have now developed systems and methods that incorporate pathway informed learning with monotherapy predictors. As is discussed in more detail below, it is generally preferred that known pathway modeling systems (preferably PARADIGM) are used to infer pathway activities from multiple cell-line data of treatment resistant and treatment sensitive cell (of the same tumor type). So developed pathway activity data are then used to build predictive models of drug response in an approach as also further discussed in more detail below (topmodel), and the top predictive model for each drug is inspected to determine which genes are often highly weighted for resistance. Those genes are then *in silico* clamped in an off-position in the known pathway modeling systems (preferably PARADIGM), and activities are re-inferred, which in effect simulates *in silico* the anticipated effect of a drug intervention *in vivo.* The topmodel is then used to reassess the newly inferred post-intervention data. As can be readily appreciated, where the reassessment indicates a shift from a prediction of drug resistance to a prediction of drug sensitivity, the simulated *in silico* intervention can be translated into a treatment recommendation for *in vivo* treatment.

In the following, the inventors have demonstrated the feasibility of such systems and methods using known breast cancer cell line data and a large panel of monotherapy drug response profiles for these cells. In order to simulate the effect of dual therapies, the inventors used the highly accurate drug response models trained upon pathway modeling system data as further described below, and inspected these pathway modeling system-based models for gene candidates that were putatively associated with resistance. These resistance-associated features were silenced *in silico* in the pathway modeling system as a proxy for simulating the effect of a targeted drug intervention against the action of those genes. The so obtained models were then used to reassess the post-intervention dataset for a shift towards sensitivity. If a shift is observed, the inference is that the drug response that the model predicted *in silico* will likely be enhanced *in vivo* by combining a first drug with a second, rationale-based targeted drug therapy against the candidate gene.

It should be appreciated that predicting the effect of a drug/feature-KO combination in this method requires highly accurate, linear classifiers. Most preferably, such classifiers use pathway modeling system data (preferably PARADIGM data) as input to allow their application without manipulation to pre-intervention and post-intervention data. In addition, linear models will also allow for inspection for feature coefficients to select resistance-associated features for simulating intervention against.

Drug Response Predictor Model Building: Predictive models promoted to use in a clinical setting must have high performance. In order to develop such a predictive model many competing models are typically generated. The performance of these multiple competing models needs to be compared to select the best performers, yet the methods to compare these performances are often not satisfactory: Typically the parameters between comparisons vary so widely that they are effectively meaningless. Some machine-learning comparison tools have been developed to manage controlling parameters. For example, software such as 'scikit-learn' and 'WEKA' are designed to very quickly gather theoretical predictive accuracies. However, to decrease runtime, such software only temporarily hold minimal representations of data in volatile memory. By their design, a new predictive algorithm must be implemented inside their software to add it to the comparison. This often necessitates laboriously translating existing code into the language of the machine-learning pipeline code (python for scikit-learn, and Java for WEKA). Comparisons to algorithms developed outside of these software tools are still extremely difficult.

To overcome at least some these difficulties, the inventors have now developed a tool ("topmodel") that decouples data management from the machine-learning algorithms applied to that data, which provides a flexible, high throughput pipeline. Topmodel reads data, performs training and validation splitting, performs all data and metadata transformations, and then writes those data to the various formats required by disparate software packages. In this way the exact same training and validation data is exposed to different algorithms implemented in different languages. Topmodel then collects results and displays them in a unified format. In short, topmodel gathers data by accessing data stored in any of the common storage formats (locally or in cloud storage services), then performs a preprocessing step in which data and metadata undergo multithreaded preprocessing, and in which the data are then written to the file formats required by individual machine-learning packages. It should be noted that this preprocessing is consistent between formats and is seeded (and therefore reproducible). In yet another step, training and evaluation is performed, with each classifier being trained on training data, and being evaluated on validation data. This is preferably performed on a cluster, increasing throughput substantially. In addition to the evaluation models, a fully-trained model is built upon the whole input dataset. In a further store and display step, each algorithm and its parameters are evaluated, and those evaluations are collected into a unified file format that can be stored in a database (queryable from a user interface). Lastly, the interface defines functions to run fully trained models on novel data, users can upload their data through the interface and receive predictions.

With respect to the data gathering step, it is noted that to build predictive models, high quality datasets with their associated metadata need to be collected. There are many collections of microarray data in the public domain. Sites like the Gene Expression Omnibus (GEO) have become the de facto data sharing depot for hundreds of large cohorts with the necessary associated metadata. There are also large-scale data-generating consortium like SU2C and TCGA which provide their own data-sharing services. However, it should be recognized that collecting these datasets requires significant effort as each storage site has their own query system, file formats, usage policies, etc. These systems are constantly being upgraded. Programmatically accessing these datasets directly is extremely fragile. Therefore, and instead of directly accessing these data-sharing repositories, topmodel is configured to read both data and metadata from any of the commonly-used formats. This includes reading tab-delimited files, BED files, accessing mySQL databases, and reading SQLite databases. Moreover, the topmodel C library can access both locally hosted databases as well as remotely hosted databases.

With respect to data preprocessing it is noted that for model performance comparisons to be commensurate, the data exposed to machine-learning packages for training should be consistent. In order to ensure data is consistent, topmodel executes all data preprocessing before exposing that data to machine-learning packages. Data preprocessing includes feature selection, data transformations, and metadata transformations, and splitting into training and validation datasets. As should be appreciated, feature selection is a common strategy for increasing robustness. Reducing the input feature-space can alleviate the 'curse of dimensionality' in which noise is modeled rather than signal. Feature selection (as opposed to feature reduction) is specifically the culling of less informative features from the current datasets. The current implementation of topmodel supports filtering by minimum variance, rank of variance, minimum information gain ratio, and information gain rank. Moreover, the inventors recognized that transforming data into a space that increases variance between subgroups of interest can boost prediction performance. Data transformations that convert to a new feature space are preferably performed prior to input to topmodel to allow features to be tracked. However, topmodel supports many data transformations that retain the original datasets feature space: discretization by sign, ranks, significance thresholds, and by Boolean expressions.

As will be readily recognized, there are many ways to interpret clinical response variables. Interpretation of clinical response variables is especially pertinent when converting continuous variables such as IC50 data into binary data (responder vs. non-responder) for use in binary classification algorithms: Multiple different thresholds for splitting may be equally rational choices. Topmodel is therefore configured to support many metadata discretization schemes, including by splitting around the median, by top-and-bottom quartiles, by sign, by ranks, by user-defined thresholds, and by Boolean expressions. There are many techniques for validating prediction robustness. Further, different prediction tasks should use different robustness metrics. For example, LOOCV is more appropriate for very small cohorts than RRS. Topmodel is therefore also configured to support many different validation methods. The technique used to measure robustness is considered a parameter in the topmodel pipeline. When taken in combination, the choices in data source, data feature selection, data transformation, and metadata transformation, and validation method, describe a large potential space of inputs. The processing time and storage needs for these preprocessing steps are significant, and topmodel therefore requires a large storage system accessible to a compute cluster. Topmodel outputs training and validation files to a hive storage system, which is large capacity and redundant. The hive is also mounted to be accessible to compute clusters, making these files directly available for training. Topmodel uses several techniques to reduce preprocessing time. Instead of downloading the dataset each time for each model, topmodel downloads data once and holds it in memory. Internal copies of the data are used to perform feature selection and transformation. These data manipulation steps are chained so that no work is repeated. Additionally, the topmodel preprocessing modules are multi-threaded. Threading allows the preprocessing steps to run concurrently, saving time, while still sharing memory, which can aid avoiding repeating work.

Preprocessing increases exponentially with the number of parameters being explored. When exploring multiple datasets with multiple feature selection methods and multiple data transformations preprocessing can become the bottleneck in the topmodel pipeline. The current multi-threaded approach can generate thousands of unique dataset manipulations in a few hours.

With respect to the training and evaluation, it should be appreciated that topmodel uses very simple 'train' and 'classify' commands to build and test models, and that all of the machine-learning packages in topmodel are run from a UNIX-like command. Supported packages must have two executables: A train command, and a classify command. The train command must receive as input at least one data file and output at least one model file. The classify command must receive as input at least one data file and one model file and output at least one results file. This is a very common schema for machine-learning algorithms that is easily supported. For example, the 'train' and 'classify' executables come out of the box for svmlight. For other algorithms that do not run from the command-line in this way, the inventors developed small wrappers. For example, glmnet models (*i.e.,* ridge-regression, lasso, and elastic-nets) are typically run from inside R so do not have a command line interface. The inventors developed two small R modules, one for training and one for classifying, that can be run from the command line using R in batch mode.

Training models: Training models is the most computationally expensive step in the topmodel pipeline. Training complex models (e.g. polynomial kernel support-vector machines) upon a dataset with thousands of features can take hours to complete on our swarm cluster nodes (quadcore Intel Xeon processors). There are at least two training jobs per model in topmodel: A set of training jobs for evaluating performance (e.g. cross-validation models), and one fully-trained model that uses the entire dataset as input. Because of the preprocessing step, training models can be completely parallelized. All models are trained on independent nodes in our cluster system. By dividing these training jobs, the time taken to generate many thousands of models is mostly restricted by the size of the cluster.

Classification: There are at least three classification jobs per model in topmodel: A set of classification jobs for evaluation on the validation dataset, a set of classification jobs for re-inspecting the training dataset, and one classification job to inspect the fully-trained model. Similarly to training, all classification steps can be run in parallel on the cluster (after training has finished). Classification uses relatively few compute-resources compared to training. Evaluation models: After all classification is complete a module in topmodel reads the results files generated by disparate machine-learning packages and converts that information into a unified reporting format. One report file is generated per model, and stored on the hive. As this is a per-model step it can also be run on the cluster. This report format describes which samples were used in training, what the raw prediction scores were from the classification algorithm, and what the accuracy of predictions was in both the training and testing cohorts. For linear models this format also includes up to 200 gene names and their coefficients in the predictive model.

Storing results: After all evaluations have been completed, a module in topmodel gathers all results into a single unified report file. This file describes all prediction tasks, feature selection methods, data transformations, metadata subgroupings, and model statistics. The topmodel module that gathers these results checks each entry for uniqueness, ensuring there is no duplication in the results. This report file acts as a file-based database of topmodel results. In a preferred aspect, another module in topmodel mirrors these topmodel results in a database that can be queried from the web. A user interface then is provided that allows display of the results queried from the database.

Prediction using topmodel: Fully-trained models can be used to predict upon novel user-submitted data. Using the topmodel user-interface, users can upload tab-delimited data for their samples. The topmodel CGI saves their data to local temporary scratch space. It then matches the features from the user data to the model being requested. Where there are missing values in the user's data null values are inserted. The requested model is then used to score the user data using a module in the topmodel C library. The scores are reported back to the topmodel user-interface in JSON format, and the user data is wiped from disk. The prediction scores in JSON format are received by the topmodel user-interface and rendered into a plot. Included in this plot is a pie-chart showing the overlap in features between the user submitted data and the model being applied. Additionally, prediction scores from the training dataset are also plotted to give context from true positive and true negative examples.

In further contemplated aspects of the inventive subject matter, and particularly in view of the above contemplated systems and methods, it should be appreciated that the systems and methods will also be suitable for identification of the mechanism of action and/or target of a new therapeutic compound. For example, multiple and distinct cells and/or tissues (typically diseased cells or tissues) are exposed to one or more candidate compounds to evaluate a potential therapeutic effect. Most typically, such effect will be measured as a GI₅₀, IC₅₀, induction of apoptosis, phenotypical change, etc. for each of the multiple and distinct cells and/or tissues, and machine learning as described herein is employed to identify one or more determinant pathway elements in the data sets of the cells and/or tissues. Such identification will readily lead to a potential target and/or mechanism of action for the new therapeutic compound. In addition, contemplated systems and methods will also be suitable to identify secondary drugs (*e.g*., known chemotherapeutic drugs) that may increase efficacy of the new therapeutic compound. Consequently, using the systems and methods described herein, it should be recognized that the mode of action and molecular targets can be identified for a new drug, as well as synergistic new drug/known drug combinations can be identified.

In the same manner, it should also be recognized that new targets for an existing drug may be identified for which no pharmaceutical compound exists. For example, where the systems and methods presented herein indicate a particular pathway element as a determinant pathway element for a successful treatment for which no current drug exists, rational drug design may be employed to develop leads and even active pharmaceutical compounds (e.g., antibodies, enzymatic inhibitors, etc.) that specifically target these so identified determinant pathway elements.

Therefore, the inventors also contemplate a method of *in silico* analysis of data sets derived from omics data of cells for identification of a drug target and/or mechanism of action. Such methods will typically include a step of informationally coupling a pathway model database to a machine learning system and a pathway analysis engine, wherein the pathway model database stores multiple and distinct data sets derived from omics data of multiple and distinct cells treated with a candidate compound (*e.g*., chemotherapeutic drug, antibody, kinase inhibitor, etc.), respectively, and wherein each data set comprises a plurality of pathway element data. A machine learning system will then receive the distinct data sets, and the machine learning system will identify a determinant pathway element in the distinct data sets that is associated with administration of the candidate compound to the cells substantially as described herein. In another step, the pathway analysis engine will receive at least one of the distinct data sets from the cells and associate the determinant pathway element in the distinct data set with a specific pathway or druggable target. The so identified specific pathway or druggable target is then used in an output (*e.g*., report file optionally with graphical representation) that correlates the candidate compound with the specific pathway or druggable target. It should also be appreciated that the method may then use the so identified new information in a manner as already described. For example, the pathway analysis engine may be used to modulate the newly identified determinant pathway element in the data set to produce a modified data set from the cell, and the machine learning system may then identify (on the basis of the modified data set) a change in a status of a treatment parameter for the cell.

### Examples

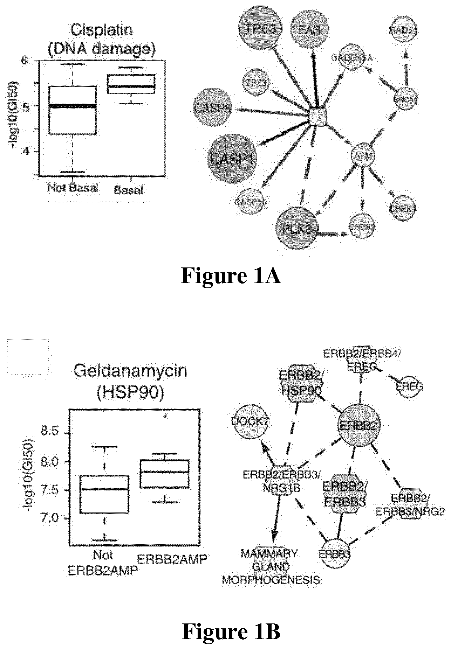

As is well known, different cell lines of a diseased tissue (*e.g.,* of breast cancer) have very different expression and regulatory environment in response to treatment with a particular drug. For example, while some types of breast cancer (*e.g*., basal, not basal) will have distinct sensitivity towards cisplatin as shown in the plot of **Figure 1A****,** other types of breast cancer (ERBB2AMP, not ERBB2AMP) will have distinct sensitivity towards Geldanamycin as shown in the plot of **Figure 1B****.** The corresponding schematic illustrations for Figs. 1A and B located to the right of the plots illustrate the corresponding exemplary pathway information for the respective cells/drug treatments where solid lines indicate transcription activation, dashed lines depict kinase activation, and a bar at the end of a line depict inhibitory effect.

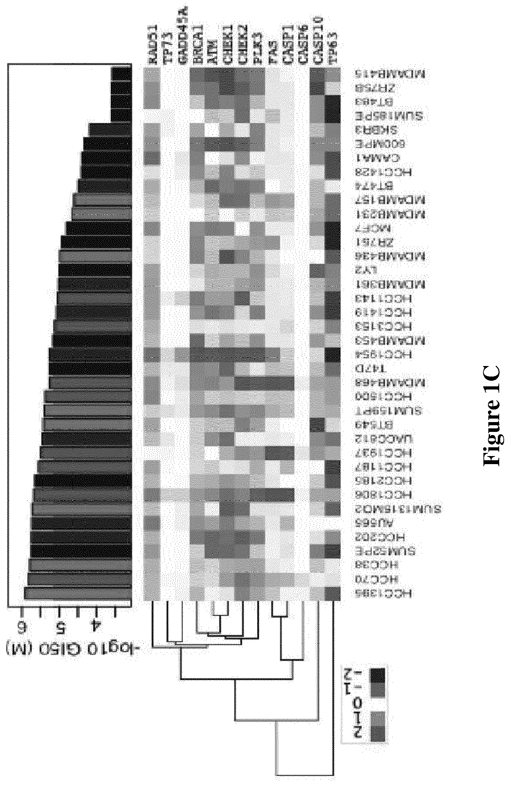

The upper panel of **Figure 1C** depicts a more detailed view of drug sensitivity of various breast cancer cell lines against cisplatin, while the lower panel shows a heat map of expression/regulation in the same cell lines (indicated at the x-axis) with respect to various target elements (indicated at the y-axis, see also schematic illustration of Fig. 1A) within a pathway of the cancer cell. As can be readily recognized, expression and gene regulation is substantially different from cell line to cell line, with no apparent pattern associated with sensitivity towards or resistance against cisplatin. Therefore, while a wealth of genomic information is available, the skilled artisan lacks effective or even informative guidance from these data to identify a suitable treatment strategy or recommendation.

For the present example, a panel of 50 breast cancer cell lines was used to provide a suitable dataset to demonstrate the effectiveness of the systems and methods (topmodel) contemplated herein. In addition to having data from several genome-wide assays, response to 138 drugs have been assayed in these cell lines. As a result, many prediction challenges can be analyzed in this dataset while holding the cohort effect constant. More specifically, Affymetrix Exon microarray expression data and Affymetrix Genome Wide SNP 6.0 microarray copy-number were obtained for 50 breast cancer cell lines and these data were used to infer pathway activities using known pathway modeling systems (as described in WO 2011/139345 and WO 2013/062505). The data that results from such transformation of expression and copy number data is a matrix of pathway-features by samples appropriate for use in systems and methods (topmodel) contemplated herein. In addition to genomics data, IC50 drug response data (GI50, Amax, ACarea, filtered ACarea, and max dose) for 138 drugs was obtained.

These data were used to build drug response classifiers (sensitive vs. resistant) in the topmodel pipeline as described in the table below. In combination these parameters describe a prospective 129,168 fully-trained models. As each model is validated by 5x3 fold cross-validation this requires training a further 15 models per fully-trained model, or 1,937,520 additional evaluation models. The total number of models to be trained is over 2 million.

| Datasets | Exon expression, SNP6 copynumber, PARADIGM |
|---|---|
| Metadatasets | 138 drug response IC50s |
| Subgroupings | median IC50, median GI50, median Amax, median ACarea, median Filtered ACarea, median max dose |
| Classifiers | NMFpredictor, SVMlight (linear kernel), SVMlight (first order polynomial kernel), SVMlight (second order polynomial kernel), WEKA SMO, WEKA j48 trees, WEKA hyperpipes, WEKA random forests, WEKA naive Bayes, WEKA JRip rules, glmnet lasso, glmnet ridge regression, glmnet elastic nets |
| Feature selection methods | None, variance ranking (20 features), variance ranking (200 features), variance ranking (2000 features) |
| Validation method | 5x3 fold cross-validation |

For the breast cancer cell line data noted above, the most accurate linear model for each drug (out of 138 available drugs) was selected for further analysis, and for each model up to 200 resistance-associated features were extracted by inspecting the coefficients in these linear models and reporting the highest ranking features. Of the 17,325 features in the pathways 5,065 were selected by at least one of the 138 drug response models as being associated with resistance. Of these 5,065 features the 200 that were associated with resistance most frequently were selected for *in silico* knock-out.

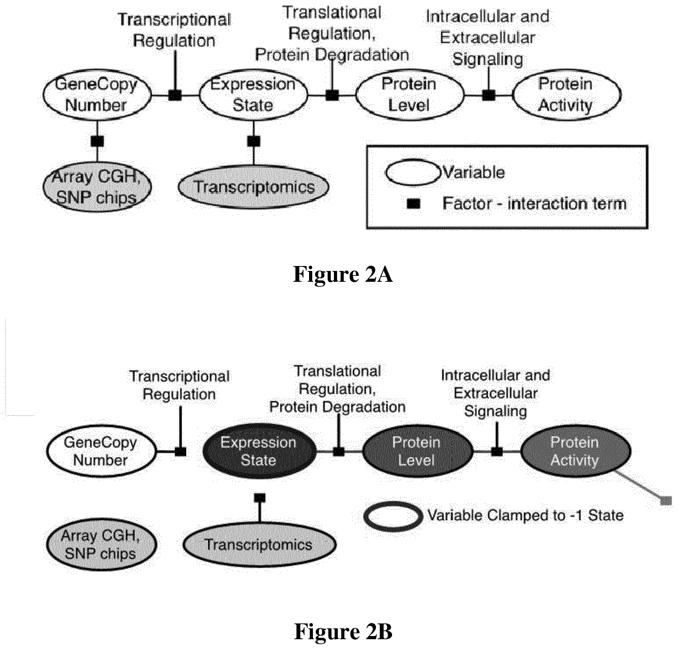

*In silico* Pathway Modulation: Preferred pathway modeling systems as described in WO 2011/139345, WO 2013/062505, and WO 2014/059036 learn inferred pathway activities by fitting observed biological data (omics data) to a central dogma module (typically based on curated *a priori* known pathway information), then allowing many modules to propagate signals to each other until they converge upon a stable state. **Figure 2A** provides a schematic illustration of a pathway model (PARADIGM) in which a gene is represented via a statistical factor graph model.

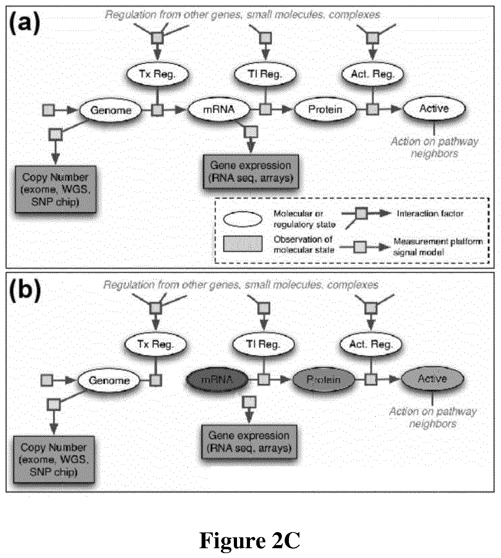

As should be readily appreciated, such pathway modeling systems can also be used to simulate the effect of a targeted intervention. For example, as schematically illustrated in **Figure 2B** for gene silencing of a gene, the target mRNA node in the central dogma module can be forced into a suppressed state, and the pathway activities re-inferred. Additionally, the knocked-down mRNA node can be disconnected from its parent nodes, which will inhibit the low mRNA state spuriously back-propagating its suppressed state to transcriptional regulators of the target gene. A further schematic example is provided in **Figure 2C** where, in panel (a) an exemplary pathway is expressed as a factor graph that advantageously allows modeling and inferring pathway activities. Evidence nodes are populated using data that are derived from genome-wide assays (typically omics data) such as expression data and copy-number data. Therefore, signals from these nodes are propagated through the factor graph. Panel (b) schematically shows an intervention simulation. In the targeted feature (knock-out of gene expression), evidence nodes are disconnected and the mRNA node is clamped to a downregulated state.

Using the above system, intervention simulations were performed for all 200 resistance associated features in the breast cancer cell lines, which generates 200 new 'post-intervention' datasets, each representing the effect of a targeted gene silencing. To quantify the effect of dual interventions, a drug-response model is applied to both the pre- and post-intervention datasets and the shift in predicted resistance is observed. The magnitude of this shift indicates how much the feature intervention synergizes with the monotherapy response that the model predicts.

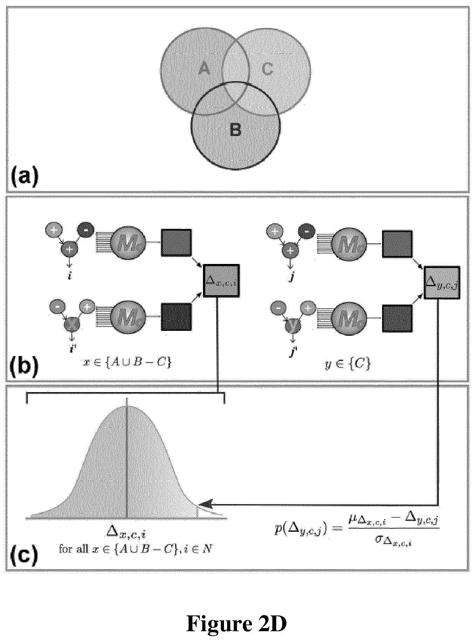

Significance Analysis And Shift Measurement: The following significance analysis was performed to further fine-tune the results. In the breast cancer example above, each linear model selected for analysis could nominate 200 features as being resistance-associated. As only the top 200 were selected from the full list of over 5,000 nominees, each linear model contained certain features that were selected and other features that were not selected. On average, a given linear model has 3 features in the 200 resistance-associated set. Thus, for any given response model there is a pool of about 197 simulated knock-down datasets that are unrelated to the model, which are used to create an empirical null distribution. Top models for each drug are then applied to all feature knock-down datasets, and those that are unrelated to the drug being analyzed create a background model with which to measure the significance of each gene that was selected as is schematically illustrated in **Figure 2D****.** Here, panel (a) schematically illustrates drug-response models A, B, & C, each containing up to 200 genes previously identified as resistance-related, and some of the genes between models A, B, & C, may overlap. When analyzing drug/feature-KO combinations from model C, all genes, x, were used from the set x ∈ {A U B - C}, in a null model. In panel (b) Model C is applied to all genes x ∈ {A U B - C} and all samples i ∈ N. The amount of shift for each feature-KO/drug/sample combination, Δ_{x,c,i} is recorded in a background model. Model C is also applied to each gene y ∈ {C}, and the amount of shift, Δ_{y,c,j} recorded. As is shown in panel (c), the amount of shift in a selected drug/gene/sample combination is then measured for significance against the background distribution from unrelated genes.

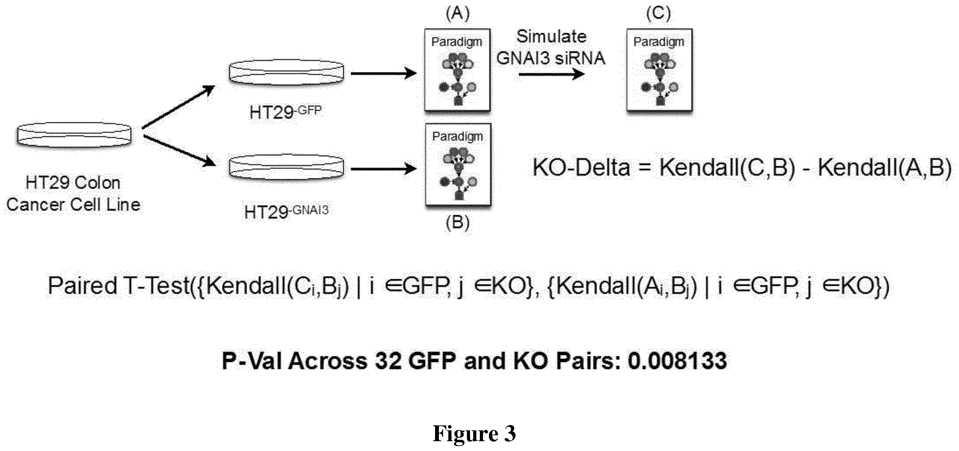

To validate such conceptual approach, the inventors used colon cancer cell line HT29 in a set of experiments as schematically shown in **Figure 3**. In a first *in vitro* experiment, an siRNA against GFP (green fluorescent protein) was expressed in the cell as negative control (as the HT29 cells do not express GFP), while in a second *in vitro* experiment, an siRNA against GNAI3 was expressed to knock down native GNAI3 expression in the cell. Omics data (gene copy number, expression level, proteomics data) were obtained for both *in vitro* experiments, and pathway analysis was performed using PARADIGM. In an independent *in silico* experiment, GNAI3 was artificially set to 'no expression', and paired T-tests were run as indicated in Figure 3 to see if the experimental conditions observed in the *in vitro* GNAI3-knock-down cells would correlate more closely to the *in silico* GNAI3-knock-down cells than the *in vitro* GFP-knock-down cells. Remarkably, the *in silico* results paralleled the *in vitro* results with a relatively high degree of statistical significance. Thus, the potential usefulness of the above approach was clearly indicated.

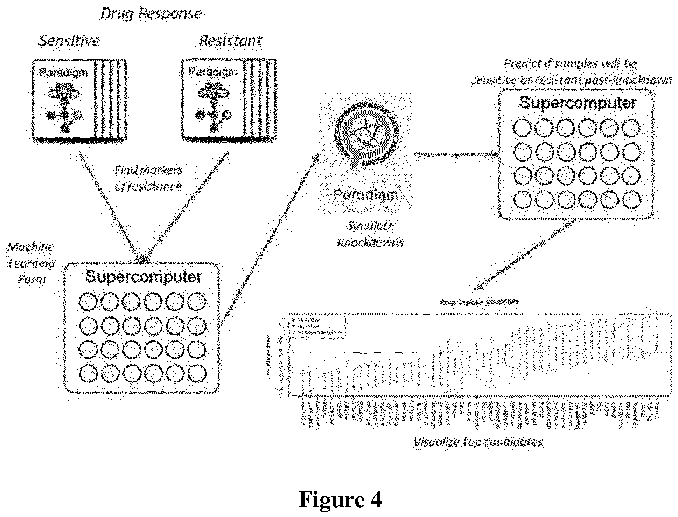

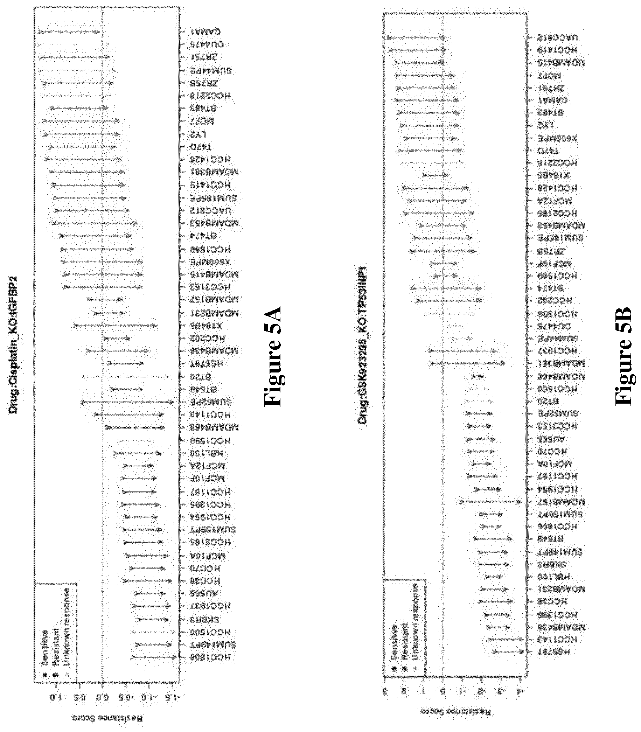

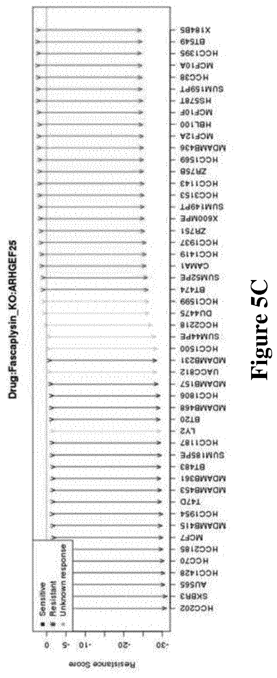

In view of the above, **Figure 4** schematically illustrates a typical embodiment of the inventive subject matter as presented herein. Here, omics data (preferably as PARADIGM data sets) of the same cell type but different drug sensitivity (*e.g.,* sensitive vs. resistant, as expressed via and on the basis of GI₅₀ values) are subjected to machine learning analysis in a machine learning farm using topmodel to so identify putative pathway elements that confer resistance and/or sensitivity towards the drug as described above. Once identified, the one or more putative pathway elements are then artificially modulated *in silico* (here: as a simulated knock-down), and the so obtained datasets are subjected to further analysis to predict whether or not (and to what degree) the modification resulted in a change in sensitivity to the drug. The results of the analysis are then provided in an output format that allows identification of pathway elements that will provide or contribute to a desired change in the drug resistance. In the example of Figure 4, the calculated/simulated change in sensitivity against cisplatin upon knock-down of IGFBP2 in breast cancer cells is indicated for each cell line using arrows. Figures 5A-5C depict predicted results for changes in drug sensitivity as a function of a calculated/simulated change in expression of a previously identified pathway element of breast cancer cells. More specifically, **Figure 5A** depicts cisplatin sensitivity and the pathway element is IGFB2, **Figure 5B** depicts GSK923295 sensitivity and the pathway element is TP53INP1, while **Figure 5C** depicts fascaplysin sensitivity and the pathway element is ARHGEF25.

Of course, it should be appreciated that the above examples only provide an illustration of the inventive subject matter and should not be deemed limiting. Indeed, while the examples provide only analysis of single pathway element modulation, it should be appreciated that multiple pathway elements may be modified, concurrently, or sequentially. Still further, it should be recognized that while knock-down changes are discussed, all modifications (*e.g*., up, down, [heterologous or otherwise recombinant] gene expression) are deemed suitable for use herein. Such modifications can be direct modifications on the nucleic acid level (*e.g.,* knock-down, knock-out, deletion, enhanced expression, enhanced stability, etc.) and/or on the protein level (*e.g.,* via antibodies, recombinant expression, injection, etc.), or indirect modifications via regulatory components (*e.g.*, by providing expression stimulators, transcription re-pressors, etc.).

Still further, it should be noted that while the above examples are used to interfere with a single pathway or pathway network, *in silico* and *in vivo* manipulations are also contemplated that affect multiple pathways, whether or not functionally associated with each other. Likewise, it should be recognized that the pathway manipulation may also be performed such that a desired outcome is artificially set, and that subsequent analysis is then performed to identify parameters that can be modified to so lead to the desired result. Moreover, while PARADIGM is a particularly preferred pathway model system, it should be appreciated that all pathway modeling systems are deemed suitable for use herein. Most typically, such modeling systems will have at least an *a priori* known component.

Thus, specific embodiments and applications of methods of drug response networks have been disclosed. It should be apparent to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

In accordance with an embodiment, it is provided a method of *in silico* analysis of data sets derived from omics data of cells, comprising:
informationally coupling a pathway model database to a machine learning system and a pathway analysis engine;
wherein the pathway model database stores a plurality of distinct data sets derived from omics data of a plurality of distinct diseased cells, respectively, and wherein each data set comprises a plurality of pathway element data;
receiving, by the machine learning system, the plurality of distinct data sets;
identifying, by the machine learning system, a determinant pathway element in the plurality of distinct data sets that is associated with a status of a treatment parameter of the diseased cells;
receiving, by the pathway analysis engine, at least one of the distinct data sets from the diseased cells;
modulating, by the pathway analysis engine, the determinant pathway element in the at least one distinct data set to produce a modified data set from the diseased cell; and identifying, by the machine learning system and using the modified data set, a change in the status of the treatment parameter for the diseased cell.

According to an embodiment, at least one of the distinct data sets is generated from a patient sample of a patient having a neoplastic disease, and wherein multiple other ones of the distinct data sets are generated from distinct cell cultures containing cells that are not from the patient, wherein preferably the method further comprising a step of generating output data that comprise a treatment recommendation for the patient.

In accordance with an embodiment, the patient has not been treated for the neoplastic disease. In accordance with an embodiment, the plurality of distinct diseased cells differ from one another with respect to sensitivity of the cells to a drug.

In accordance with an embodiment, a first set of the plurality of distinct diseased cells are sensitive to treatment with a drug, and wherein a second set of the plurality of distinct diseased cells are resistant to treatment with the drug.

In accordance with an embodiment, the method is further comprising a step of identifying a drug that targets the determinant pathway element when the change in status exceeds a predetermined threshold.

In accordance with an embodiment, the omics data are selected from the group consisting of gene copy number data, gene mutation data, gene methylation data, gene expression data, RNA splice information data, siRNA data, RNA translation data, and protein activity data.

According to a further embodiment the distinct data sets are PARADIGM datasets.

In accordance with an embodiment the determinant pathway element is an expression state of a gene, a protein level of a protein, and/or a protein activity of a protein.

In accordance with an embodiment, the treatment parameter is treatment with a drug, and wherein the status is sensitivity to the drug or resistance to the drug.

In accordance with an embodiment, the change in status is a change from resistance to the drug to sensitivity to the drug.

In accordance with an embodiment, the method is further comprising a step of pre-processing the datasets that includes feature selection, data transformation, metadata transformation, and/or splitting into training and validation datasets.

According to an embodiment, it is further provided a system for *in silico* analysis of data sets derived from omics data of cells, comprising:
a pathway model database informationally coupled to a machine learning system and a pathway analysis engine;
wherein the pathway model database is programmed to store a plurality of distinct data sets derived from omics data of a plurality of distinct diseased cells, respectively, and wherein each data set comprises a plurality of pathway element data;
wherein the machine learning system is programmed to receive from the pathway model database the plurality of distinct data sets, and wherein the machine learning system is further programmed to identify a determinant pathway element in the plurality of distinct data sets that is associated with a status of a treatment parameter of the diseased cells;
wherein the pathway analysis engine is programmed to receive at least one of the distinct data sets from the diseased cells and further programmed to modulate the determinant pathway element in the at least one distinct data set to produce a modified data set from the diseased cell; and
wherein the machine learning system is programmed to identify a change in the status of the treatment parameter for the diseased cell using the modified data set.

Preferably at least one of the distinct data sets is generated from a patient sample of a patient having a neoplastic disease, and wherein multiple other ones of the distinct data sets are generated from distinct cell cultures containing cells that are not from the patient.

In accordance with an embodiment, the patient has not been treated for the neoplastic disease. In accordance with an embodiment, the machine learning system is programmed to generate output data that comprise a treatment recommendation for the patient.

In accordance with an embodiment, it is further provided a non-transient computer readable medium containing program instructions for causing a computer system in which a pathway model database is coupled to a machine learning system and a pathway analysis engine to perform a method comprising the steps of:
transferring from the pathway model database to the machine learning system a plurality of distinct data sets derived from omics data of a plurality of distinct diseased cells, respectively, and wherein each data set comprises a plurality of pathway element data;
identifying, by the machine learning system, a determinant pathway element in the plurality of distinct data sets that is associated with a status of a treatment parameter of the diseased cells;
receiving, by the pathway analysis engine, at least one of the distinct data sets from the diseased cells;
modulating, by the pathway analysis engine, the determinant pathway element in the at least one distinct data set to produce a modified data set from the diseased cell; and identifying, by the machine learning system and using the modified data set, a change in the status of the treatment parameter for the diseased cell.

Preferably the omics data are selected from the group consisting of gene copy number data, gene mutation data, gene methylation data, gene expression data, RNA splice information data, siRNA data, RNA translation data, and protein activity data.

In accordance with an embodiment, the distinct data sets are PARADIGM datasets.

In accordance with an embodiment, it is further provided a method of *in silico* analysis of data sets derived from omics data of cells, comprising:
informationally coupling a pathway model database to a machine learning system and a pathway analysis engine;
wherein the pathway model database stores a plurality of distinct data sets derived from omics data of a plurality of distinct cells treated with a candidate compound, respectively, and wherein each data set comprises a plurality of pathway element data;
receiving, by the machine learning system, the plurality of distinct data sets;
identifying, by the machine learning system, a determinant pathway element in the plurality of distinct data sets that is associated with administration of the candidate compound to the cells;
receiving, by the pathway analysis engine, at least one of the distinct data sets from the cells; associating, by the pathway analysis engine, the determinant pathway element in the at least one distinct data set with a specific pathway or druggable target, and producing an output that correlates the candidate compound with the specific pathway or druggable target.

Preferably the candidate compound is a chemotherapeutic drug.

In accordance with an embodiment, the method is further comprising a step of modulating, by the pathway analysis engine, the determinant pathway element in the at least one distinct data set to produce a modified data set from the cell, and a further step of identifying, by the machine learning system and using the modified data set, a change in a status of a treatment parameter for the cell.

## Claims

1. A method of *in silico* analysis of data sets derived from omics data of cells, comprising:
- informationally coupling a pathway model database to a machine learning system and a pathway analysis engine;
- generating or obtaining at least one distinct data set from a patient sample comprising a diseased cell or from a patient having a neoplastic disease, and
- generating or obtaining multiple other distinct data sets from distinct cell cultures containing cells that are not from the patient to produce a plurality of distinct data sets, wherein each data set comprises a plurality of pathway element data;
wherein the pathway model database stores the data sets;
- receiving, by the machine learning system, the data sets;
- identifying, by the machine learning system, a determinant pathway element in the data set that is associated with at least one of sensitivity or resistance of the patient sample to a treatment;
- receiving, by the pathway analysis engine, the data set;
- modifying *in silico,* by the pathway analysis engine, the determinant pathway element in the data set to produce a modified data set from the patient sample, wherein the modified data set comprises the determinant pathway element that is modified directly on either a nucleic acid level, a protein level, or indirectly via regulatory component, from a first state to a second state; and
- identifying, by the machine learning system and using the modified data set, an effect of the modified data set in a drug intervention *in vivo.*

2. The method of claim 1 wherein the pathway model database further stores a plurality of distinct data sets derived from omics data of a plurality of distinct diseased cells that are not from the patient, respectively.

3. The method of claim 2 wherein the distinct diseased cells that are not from the patient are of the same neoplastic type as the neoplastic disease of the patient.

4. The method of claim 2 wherein the plurality of distinct diseased cells differ from one another with respect to sensitivity of the cells to a drug.

5. The method of claim 4 wherein a first set of the plurality of distinct diseased cells are sensitive to treatment with a drug, and wherein a second set of the plurality of distinct diseased cells are resistant to treatment with the drug.

6. The method of claim 1 further comprising a step of generating output data that comprise a treatment recommendation for the patient.

7. The method of claim 1 further comprising a step of identifying a drug that targets the determinant pathway element when the change in status exceeds a predetermined threshold.

8. The method of claim 1 wherein the omics data are selected from the group consisting of gene copy number data, gene mutation data, gene methylation data, gene expression data, RNA splice information data, siRNA data, RNA translation data, and protein activity data.

9. The method of claim 1 wherein the data set is a PARADIGM dataset.

10. The method of claim 1 wherein the determinant pathway element is an expression state of a gene, a protein level of a protein, and/or a protein activity of a protein.

11. The method of claim 1 wherein the data set that is further associated with a change from resistance to the drug to sensitivity to the drug.

12. The method of claim 1 further comprising a step of pre-processing the datasets that includes feature selection, data transformation, metadata transformation, and/or splitting into training and validation datasets.

13. A system for *in silico* analysis of data sets derived from omics data of cells, comprising:
a pathway model database informationally coupled to a machine learning system and a pathway analysis engine;
wherein the pathway model database is programmed to generate or obtain at least one distinct data set from a patient sample comprising a diseased cell or from a patient having a neoplastic disease, and is further programmed to generate or obtain multiple other distinct data sets from distinct cell cultures containing cells that are not from the patient to produce a plurality of distinct data sets, wherein each data set comprises a plurality of pathway element data;
wherein the machine learning system is programmed to receive from the pathway model database the data sets, and wherein the machine learning system is further programmed to identify a determinant pathway element in the at least one distinct data set that is associated with at least one of sensitivity or resistance of the patient sample to a treatment;
wherein the pathway analysis engine is programmed to receive the data set and is further programmed to simulate *in silico,* the determinant pathway element in the at least one distinct data set to produce a modified data set from the patient sample, wherein the modified data set comprises the determinant pathway element that is modified directly on at least one of a nucleic acid level, a protein level, or indirectly via regulatory component, from a first state to a second state; and
wherein the machine learning system is programmed to identify an effect of the modified data set in a drug intervention in vivo using the modified data set.

14. The system of claim 13 wherein the machine learning system is programmed to generate output data that comprise a treatment recommendation for the patient.

15. A non-transient computer readable medium containing program instructions for causing a computer system in which a pathway model database is coupled to a machine learning system and a pathway analysis engine to perform a method comprising the steps of:
- obtaining at least one distinct data set from a patient sample comprising a diseased cell or from a patient having a neoplastic disease, and
- obtaining multiple other distinct data sets from distinct cell cultures containing cells that are not from the patient to produce a plurality of distinct data sets, wherein each data set comprises a plurality of pathway element data;
- transferring from the pathway model database to the machine learning system the data set derived from omics data of a patient sample of a patient having a neoplastic disease, wherein the data set comprises a plurality of pathway element data;
- identifying, by the machine learning system, a determinant pathway element in the data set that is associated with at least one of sensitivity or resistance of the patient sample to a treatment;
- receiving, by the pathway analysis engine, the data set from the diseased cells;
- modifying *in silico,* by the pathway analysis engine, the determinant pathway element in the data set to produce a modified data set from the patient sample, wherein the modified data set comprises the determinant pathway element that is modified directly on at least one of a nucleic acid level, a protein level, or indirectly via regulatory component, from a first state to a second state; and
- identifying, by the machine learning system and using the modified data set, an effect of the modified data set in a drug intervention *in vivo.*
